# EUROPÄISCHE PATENTSCHRIFT

(11) **EP 0 765 343 B1**
(45) Veröffentlichungstag und Bekanntmachung des Hinweises auf die Patenterteilung: **15.11.2000**
(21) Anmeldenummer: 95922496.5
(22) Anmeldetag: 02.06.1995
(51) Int. Cl.: C07K 16/28, C12N 5/20, G01N 33/577, A61K 39/395, A61K 49/00

(54) **MONOKLONALER ANTIKÖRPER GEGEN CD44v6**
MONOCLONAL ANTIBODY ACTIVE AGAINST CD44v6
ANTICORPS MONOCLONAL ACTIF CONTRE LE CD44V6

(30) Priorität: 08.06.1994 DE 4419913; 02.09.1994 DE 4431297
(43) Veröffentlichungstag der Anmeldung: 02.04.1997
(73) Patentinhaber: Boehringer Ingelheim International GmbH, 55218 Ingelheim am Rhein (DE); Forschungszentrum Karlsruhe GmbH, 76133 Karlsruhe (DE)
(72) Erfinder: ADOLF, Günther, R., A-1070 Wien (AT); PATZELT, Erik, A-3002 Punkersdorf (AT)
(86) Internationale Anmeldenummer: EP9502126
(87) Internationale Veröffentlichungsnummer: WO9533771

(56) Entgegenhaltungen:
- WO-A-94/12631
- WO-A-95/00658
- THE JOURNAL OF CELL BIOLOGY, Bd. 122, Nr. 2, Juli 1993 NEW YORK, NY, VSA, Seiten 431-442, M. SALMI ET AL. 'Regulated expression of exon v6 containing isoforms of CD44 in man: Downregulation during malignant transformation of tumors of squamocellular origin.'
- '8th INTERNATIONAL CONGRESS OF IMMUNOLOGY, Budapest, 23-28 August 1992' August 1992 , SPRINGER VERLAG , BUDAPEST, UNGARN siehe M. SALMI et al.:'Expression of domain 3 containing isoforms of CD44 in man' (W-47/I-34).
- INTERNATIONAL JOURNAL OF CANCER, Bd. supplement 8, 1994 GENF, SCHWEIZ, Seiten 110-115, D. JACKSON ET AL. 'Expression of alternatively spliced forms of the CD44 extracellular-matrix receptor on human lung carcinomas.'
- CANCER RESEARCH, Bd. 53, Nr. 18, 15.September 1993 BALTIMORE, MD, VSA, Seiten 4197-4203, K. HEIDER ET AL. 'Differential expression of CD44 splice variants in intestinal- and diffuse-type human gastric carcinomas and normal gastric mucosa.' in der Anmeldung erwähnt
- CANCER RESEARCH, Bd. 53, Nr. 20, 15.Oktober 1993 BALTIMORE, MD, VSA, Seiten 4754-4756, V. WIELENGA ET AL. 'Expression of CD44 variant proteins in human colorectal cancer is related to tumor progression.' in der Anmeldung erwähnt
- CANCER RESEARCH, Bd. 53, Nr. 4, 15.Februar 1993 BALTIMORE, MD, VSA, Seiten 819-825, R. KREITMAN ET AL. 'Pseudomonas exotoxin-based immunotoxins containing the antibody LL2 or LL2-Fab' induce regression of subcutaneous human B-cell lymphoma in mice.' in der Anmeldung erwähnt

## Beschreibung

Die Erfindung betrifft einen monoklonalen Antikörper gegen ein Epitop, das vom varianten Exon v6 des CD44-Gens kodiert wird, davon abgeleitete Antikörpermoleküle sowie Verwendungen des Antikörpers bzw. der Antikörpermoleküle in Diagnostik und Therapie.

Es wurde kürzlich gezeigt, daß die Expression von Varianten des Oberflächen-Glykoproteins CD44 notwendig und hinreichend ist, um sogenanntes spontanes metastatisches Verhalten sowohl in einer nicht-metastasierenden Pankreas-Adenokarzinom-Zellinie der Ratte als auch in einer nicht-metastasierenden Fibrosarkom-Zellinie der Ratte auszulösen (Günthert *et al*., 1991). Während die kleinste CD44-Isoform, die Standardform CD44s (oder CD44std), in einer Reihe verschiedener Gewebe, darunter Epithelzellen, ubiquitär exprimiert wird, werden bestimmte Spleißvarianten von CD44 (CD44v, CD44var) nur auf einer Untergruppe von Epithelzellen exprimiert. Die CD44-Varianten werden durch alternatives Spleißen so erzeugt, daß die Sequenzen von 10 Exons (v1-v10) in CD44s komplett ausgeschnitten werden, jedoch in den größeren Varianten in verschiedenen Kombinationen vorkommen können (Screaton *et al*., 1992; Tölg *et al*., 1993; Hofmann *et al*., 1991). Die Varianten unterscheiden sich dadurch, daß an einer bestimmten Stelle des extrazellulären Teils des Proteins unterschiedliche Aminosäuresequenzen inseriert sind. Solche Varianten konnten in verschiedenen menschlichen Tumorzellen und in menschlichem Tumorgewebe nachgewiesen werden. So wurde kürzlich die Expression von CD44-Varianten im Verlauf der kolorektalen Karzinogenese untersucht (Heider *et al*., 1993a). Die Expression von CD44-Varianten fehlt in normalem menschlichen Kolonepithel, und nur eine schwache Expression ist in den proliferierenden Zellen der Krypten nachweisbar. In späteren Stadien der Tumorprogression, z.B. in Adenokarzinomen, exprimieren alle malignen Entartungen Varianten von CD44. Gewebsexpression von variantem CD44 auf hohem Niveau konnte auch in aggressiven Non-Hodgkin-Lymphomen gezeigt werden (Koopman *et al*., 1993).

Eine besondere Rolle insbesondere bei der metastatischen Ausbreitung scheint das Exon v6 zu spielen (Rudy *et al*., 1993). Im Tiermodell konnten Antikörper gegen v6-spezifische Epitope die Ansiedlung metastasierender Zellen und das Wachstum von Metastasen verhindern (Seiter *et al*., 1993). In Kolonkarzinomen korreliert v6-Expression mit der Tumorprogression (Wielenga *et al*., 1993). In Magenkarzinomen ist die v6-Expression ein wichtiger diagnostischer Marker bei der Differenzierung zwischen Tumoren vom intestinalen und solchen vom diffusen Typ (Heider *et al*., 1993b). Die v6-Expression wurde in den beiden letztgenannten Arbeiten mit Hilfe von Antikörpern gegen v6-spezifische Epitope gemessen.

Monoklonale Antikörper gegen Epitope, die vom Exon v6 kodiert werden, sind im Stand der Technik bekannt (Hofmann *et al*., 1991; Wielenga *et al*., 1993). Wegen des hohen potentiellen Nutzens, den solche Antikörper in Diagnose und Therapie haben können, besteht ein hoher Bedarf an Antikörpern mit verbesserten Eigenschaften.

Salmi *et al*., (J. Cell Biol. 122: 431-442, 1993 sowie '8^{th} Int. Congress of Immunol., Budapest, 23-28 Aug. 1992, Springer Verlag, Budapest, Ungarn) beschreiben die Herstellung des v6-spezifischen Antikörpers Var3.1, der an das Peptid STTEETATQKEQWFGN bindet. Jackson *et al*. (Int. J. Cancer Supplement 8: 110-15, 1994) beschreiben Experimente mit den v6-spezifischen Antikörpern 2F10 und 8G5, ohne deren Epitop anzugeben.

Aufgabe der vorliegenden Erfindung war es, einen Antikörper bereitzustellen, der gegenüber den bekannten v6-spezifischen Antikörpern signifikant bessere Eigenschaften aufweist.

Diese Aufgabe konnte mit der vorliegenden Erfindung gelöst werden. Sie betrifft einen Antikörper mit der Bezeichnung VFF-18. Die Erfindung betrifft ferner eine Hybridoma-Zellinie, die diesen Antikörper sezerniert, und die unter der Hinterlegungsnummer DSM ACC2174 bei der DSM-Deutsche Sammlung für Mikroorganismen und Zellkulturen GmbH, Mascheroder Weg 1b, D-38 124 Braunschweig, Deutschland, hinterlegt wurde.

Im folgenden werden unter den Begriffen Antikörper oder Antikörpermoleküle nicht nur komplette Immunglobuline verstanden, sondern auch im Hinblick auf Bindungsspezifität und -affinität äquivalente Substanzen wie die beschriebenen Antikörper-Derivate und rekombinanten Antikörpermoleküle.

Der erfindungsgemäße Antikörper VFF-18 wurde mit einem Verfahren gemäß Beispiel 1 hergestellt. Der Antikörper kann ferner aus der hinterlegten Hybridomzellinie erhalten werden. Es liegt im Können des Durchschnittsfachmanns, Derivate des erfindungsgemäßen Antikörpers herzustellen oder, ausgehend von einer Sequenzanalyse dieses Antikörpers und/oder unter Verwendung der Hybridoma-Zellinie, die diesen Antikörper produziert, rekombinante Antikörpermoleküle mit dem gleichen Idiotyp zu erzeugen, d.h. Antikörpermoleküle, die im Bereich der Antigen-Bindungsstelle (*complementarity-determining regions*, CDR) die gleiche Aminosäuresequenz aufweisen wie der Antikörper VFF-18. Solche Derivate und rekombinanten Antikörpermoleküle sind daher ausdrücklich in die Erfindung eingeschlossen.

Aus dem kompletten Immunglobulin des VFF-18-Antikörpers können beispielsweise Fab- oder F(ab')₂-Fragmente oder andere Fragmente erzeugt werden (Kreitman *et al*., 1993). Für diagnostische Verfahren können VFF-18-Antikörpermoleküle, Fragmente davon oder rekombinante Antikörpermoleküle mit dem gleichen Idiotyp beispielsweise mit radioaktiven Isotopen wie ¹³¹I, ¹¹¹In, ^{99m}Tc oder radioaktiven Verbindungen (Larson *et al*., 1991; Thomas *et al*., 1989; Srivastava, 1988), Enzymen wie Peroxidase oder alkalischer Phosphatase (Catty *et* Raykundalia, 1989), mit Fluoreszenzfarbstoffen (Johnson, 1989) oder Biotinmolekülen (Guesdon *et al*., 1979) verknüpft werden. Für therapeutische Anwendungen können VFF-18 oder VFF-18-abgeleitete Antikörpermoleküle mit Toxinen (Vitetta *et* *al*., 1991; Vitetta *et* Thorpe, 1991; Kreitman *et al*., 1993; Theuer *et al*., 1993), Zytostatika (Schrappe *et al*., 1992), Prodrugs (Wang *et al*., 1992; Senter *et al*., 1989) oder radioaktiven Substanzen verknüpft werden. Der Antikörper kann ferner mit einem Zytokin oder einem anderen immunmodulatorischen Polypeptid verknüpft sein, z.B. mit Tumornekrosefaktor oder Interleukin-2.

Der Fachmann ist weiterhin in der Lage, nach Analyse der Aminosäuresequenz des Antikörpers VFF-18 und/oder unter Verwendung der Hybridoma-Zellinie, die diesen Antikörper produziert, insbesondere der darin enthaltenen genetischen Information, rekombinante Antikörpermoleküle mit dem gleichen Idiotyp wie VFF-18 herzustellen. Entsprechende Verfahren sind Stand der Technik. Solche rekombinanten Antikörpermoleküle können z.B. humanisierte Antikörper (Shin *et al*., 1989; Güssow *et* Seemann, 1991), bispezifische Antikörper (Weiner *et al*., 1993; Goodwin, 1989), *single-chain*-Antikörper (scFv, Johnson *et* Bird, 1991), komplette oder fragmentarische Immunglobuline (Coloma *et al*., 1992; Nesbit *et al*., 1992; Barbas *et al*., 1992), oder durch *chain shuffling* erzeugte Antikörper (Winter *et al*., 1994) sein. Humanisierte Antikörper können beispielsweise durch CDR-grafting (EP 0239400) hergestellt werden. Auch Framework-Regionen können modifiziert werden (EP 0519596). Zur Humanisierung von Antikörpern können heute Methoden wie PCR (s. z.B. EP 0368684; EP 0438310; WO 9207075) oder Computer-modelling (s. z.B. WO 9222653) angewendet werden. Es können auch Fusionsproteine, beispielsweise *single-chain*-Antikörper/Toxin-Fusionsproteine (Chaudhary *et al*., 1990; Friedman *et al*., 1993) hergestellt werden. Solche Antikörpermoleküle sind daher ebenfalls in der Erfindung mit eingeschlossen.

Es liegt ebenfalls im Bereich des Könnens des Durchschnittsfachmanns, das exakte Epitop von VFF-18 zu ermitteln und mit dessen Kenntnis äquivalente Antikörper mit der gleichen Bindungsspezifität herzustellen. Dies kann beispielsweise durch Peptidbindungsstudien wie in Beispiel 2 geschehen, etwa durch Variation des Peptids Hu1. Solche Antikörper sind daher ebenfalls in der Erfindung eingeschlossen.

Ein weiterer Aspekt der Erfindung ist die Verwendung von VFF-18 oder VFF-18-abgeleiteten oder äquivalenten Antikörpermolekülen in Diagnostik und Therapie.

Diagnostische Verfahren können an sich bekannte Verfahren unter Verwendung der erfindungsgemäßen Antikörpermoleküle sein, z.B. Enzym-gekoppelte Immunoassays (ELISA, Catty *et* Raykundalia, 1989), Radioimmunoassays (Catty *et* Murphy, 1989), immunhistochemische Verfahren (Heider *et al*., 1993b) oder Western Blots. Solche Verfahren können zweckmäßig mit aus dem Körper entnommenen Gewebeproben oder Körperflüssigkeiten, beispielsweise aus Biopsien, durchgeführt werden. Die Untersuchungen können qualitativ, semiquantitativ oder quantitativ erfolgen. Der Antikörper oder die Antikörpermoleküle können dabei eingesetzt werden, wie es beispielweise im Stand der Technik für andere v6-spezifische Antikörper beschrieben wurde (WO 9500851), wobei die vorteilhaften Eigenschaften des erfindungsgemäßen Antikörpers oder der erfindungsgemäßen Antikörpermoleküle eine signifikante Verbesserung solcher Verfahren bedeuten.

Neben der *in-vitro*-Diagnostik eignen sich die erfindungsgemäßen Antikörpermoleküle auch zur *in-vivo*-Diagnostik, insbesondere von Tumoren. Trägt das Antikörpermolekül ein detektierbares Label, kann eine Detektion des Labels zu diagnostischen Zwecken, z.B. Visualisierung des Tumors *in vivo* (Imaging), oder beispielsweise zur radiounterstützten Chirurgie (*radioguided surgery*) erfolgen. Für die Verwendung von mit radioaktiven Isotopen konjugierten Antikörpern zur Immunszintigraphie (Imaging) beispielsweise gibt es eine Reihe von Protokollen, auf deren Grundlage der Fachmann die Erfindung ausführen kann (Siccardi *et al*., 1989; Keenan *et al*., 1987; Perkins *et* Pimm, 1992; Colcher *et al*., 1987; Thompson *et al*., 1984).

Eine therapeutische Anwendung kann beispielsweise analog zu der Verwendung des Antikörpers 1.1ASML (Seiter *et al*., 1993) erfolgen. Dabei kann die Applikation systemisch oder topisch erfolgen, beispielsweise durch intravenöse (als Bolus oder Dauerinfusion), intraperitoneale, intramuskuläre, subkutane o.a. Injektion/Infusion. Es können auch einzelne Organe oder Glieder perfundiert werden. Protokolle für die Verabreichung von konjugierten oder nichtkonjugerten Antikörpern (sei es als komplette Immunglobuline, Fragmente, rekombinante chimäre Moleküle o.ä.) sind Stand der Technik (Mulshine *et al*., 1991; Larson *et al*., 1991; Vitetta *et* Thorpe, 1991; Vitetta *et al*., 1991; Breitz *et al*., 1992; Press *et al*., 1989; Weiner *et al*., 1989; Chatal *et al*., 1989; Sears *et al*., 1982).

Die überlegenen Eigenschaften von VFF-18 gegenüber anderen anti-CD44v6-Antikörpern zeigen die Beispiele 2 bis 4.

### Abbildungen

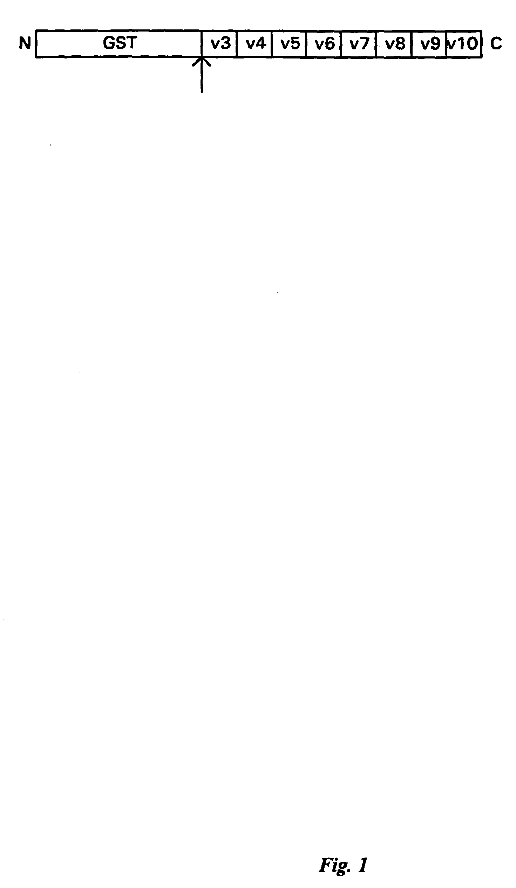

***Figur 1:*** Schematische Darstellung des GST-CD44(v3-v10)-Fusionsproteins. GST = Glutathion-S-transferase von *Schistosoma japonicum*. v3-v10 = variante Insertion von Keratinozyten-CD44. Der Pfeil markiert eine Thrombin-Spaltstelle.

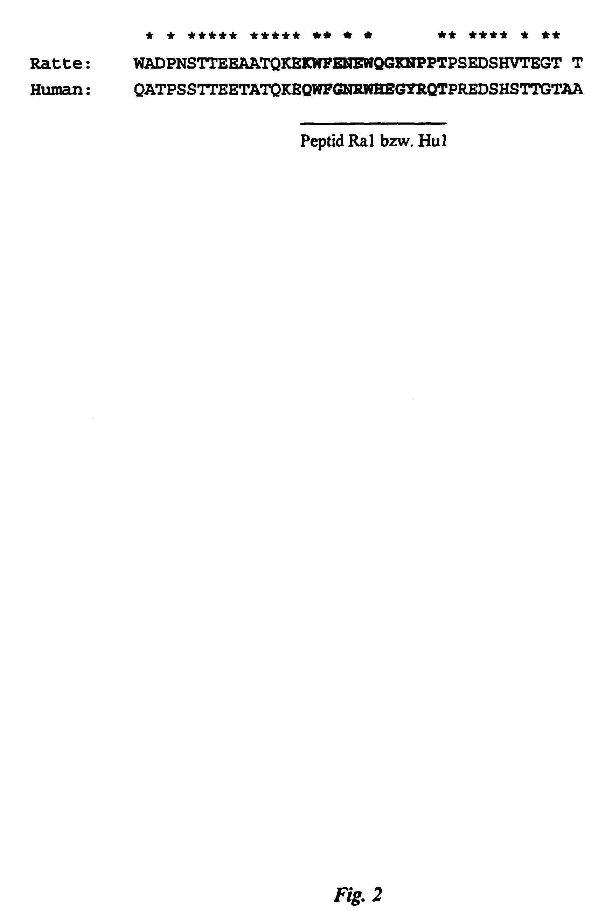

***Figur 2:*** Sequenzvergleich von Exon v6 des CD44-Gens zwischen Mensch und Ratte. Die Sequenzen der Peptide Ra1 und Hu1, an die die Antikörper 1.1ASML (anti-Ratte CD44v6) und VFF-18 (anti-human CD44v6) binden, sind fett hervorgehoben. Identische Aminosäuren sind durch einen Stern gekennzeichnet.

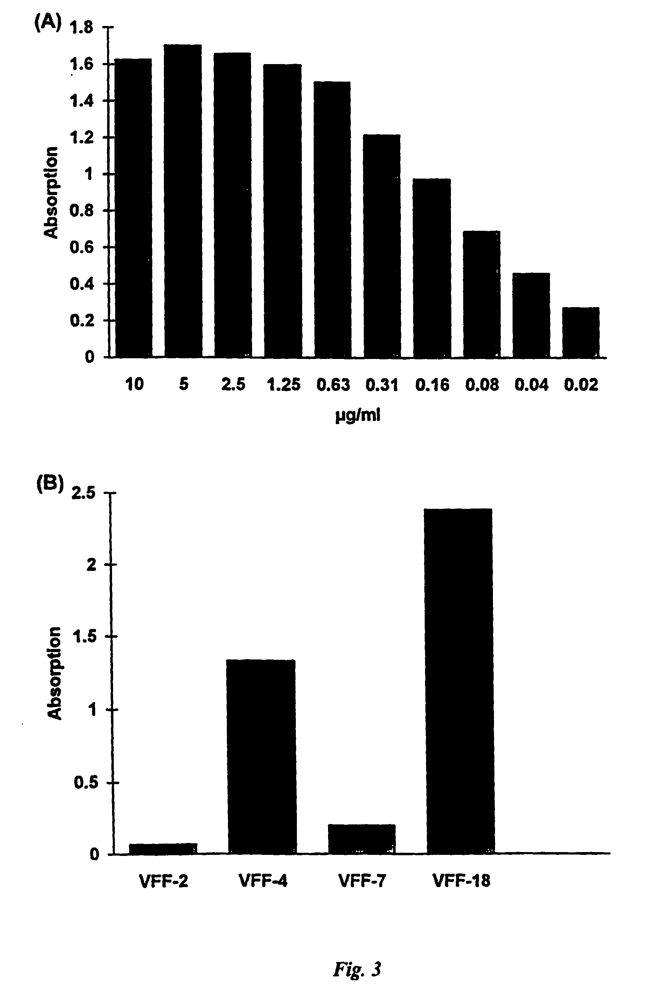

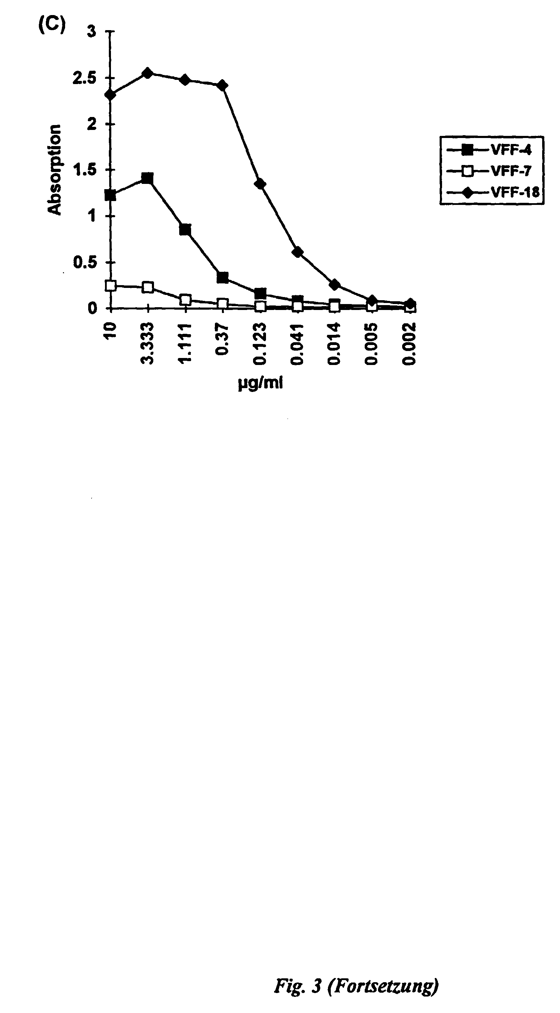

***Figur 3:*** Bindung von CD44v6- spezifischen Antikörpern an synthetische Peptide. Die Bindung der Antikörper an die Peptide wurde in einen ELISA bestimmt, bei dem die Peptide immobilisiert und dann mit Antikörperlösungen inkubiert wurden. Nach entsprechenden Waschschritten wurde gebundener Antikörper mit Peroxidase-konjugiertem anti-Maus-IgG-Antikörper nachgewiesen. ***(A)***: Im ersten Experiment wurde die Bindung des für Ratten-CD44v6 spezifischen Antikörpers 1.1ASML an das Peptid Ra1 (KWFEN EWQGK NPPT) nachgewiesen. ***(B)***: In einem weiteren Versuch wurde ein zu Ra1 homologes Peptid aus der humanen CD44v6-Sequenz synthetisiert. Verschiedene anti-human-CD44v6-Hybridomüberstände wurden an dieses Peptid Hu1 (QWFGN RWHEG YRQT) gebunden. Dabei zeigt sich, daß VFF-18 wesentlich besser an das Peptid bindet als die übrigen eingesetzten Antikörper. ***(C)***: Für eine quantitative Evaluierung wurde dieses Experiment mit verschiedenen Konzentrationen von gereinigten Antikörpern wiederholt. Auch hier zeigt sich, daß VFF-18 eine höhere Bindungsaffinität als die anderen Antikörper aufweist.

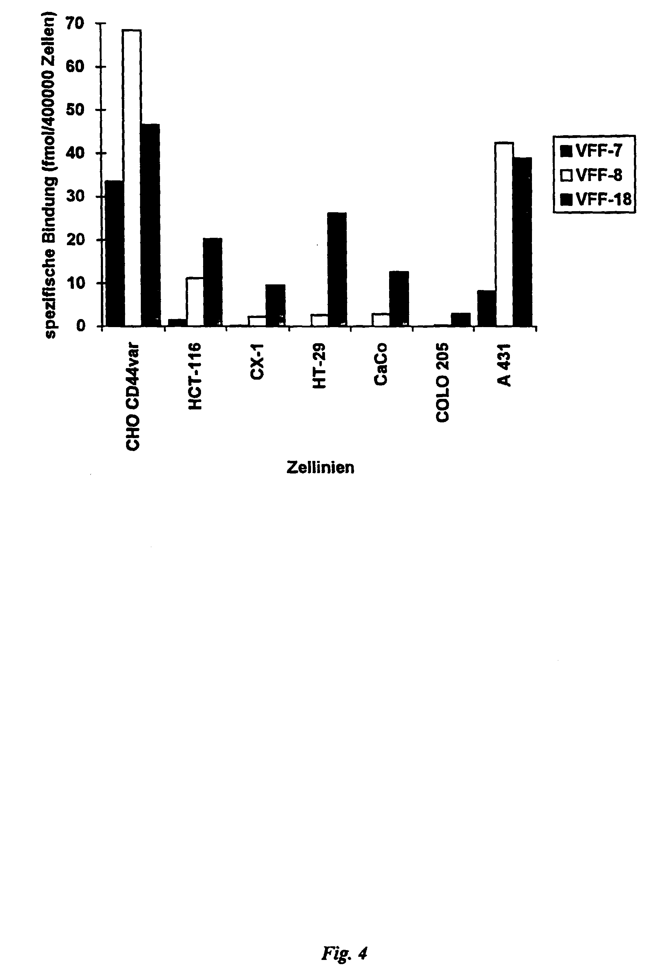

***Figur 4:*** Bindung radioaktiv markierter Antikörper an Tumorzellen. Die drei Antikörper VFF-7 (anti-v6), VFF-8 (anti-v5) und VFF-18 (anti-v6) wurden mit N-Succinimyl[2,3-³H]propionat radioaktiv markiert und für Bindungstests an verschiedenen Tumorzellinien eingesetzt. Folgende Zellinien wurden dabei verwendet: CHO-CD44var: rekombinante Hamsterzellinie (Chinese Hamster Ovary), die humanes variantes CD44 (Exons v3-v10) an der Oberfläche exprimiert; HCT-116, CX-1, HT-29, CaCo, COLO 205: humane Kolonkarzinom-Linien, A431: humane Plattenepithelkarzinom-Linie. Gezeigt ist die spezifische Bindung der Antikörper an die verschiedenen Zellinien. Während die Bindung der v6-spezifischen Antikörper VFF-7 und VFF-18 an die rekombinante Zellinie CHO-CD44var in der gleichen Größenordnung liegt, zeigen die Antikörper an den Tumorzellinien sehr unterschiedliches Bindungsverhalten. In einigen Fällen beobachtet man nur VFF-18- und in geringerem Umfang VFF-8-Bindung (HT-29, CaCo, COLO 205), bei den anderen Zellinien bindet VFF-18 wesentlich besser als VFF-7.

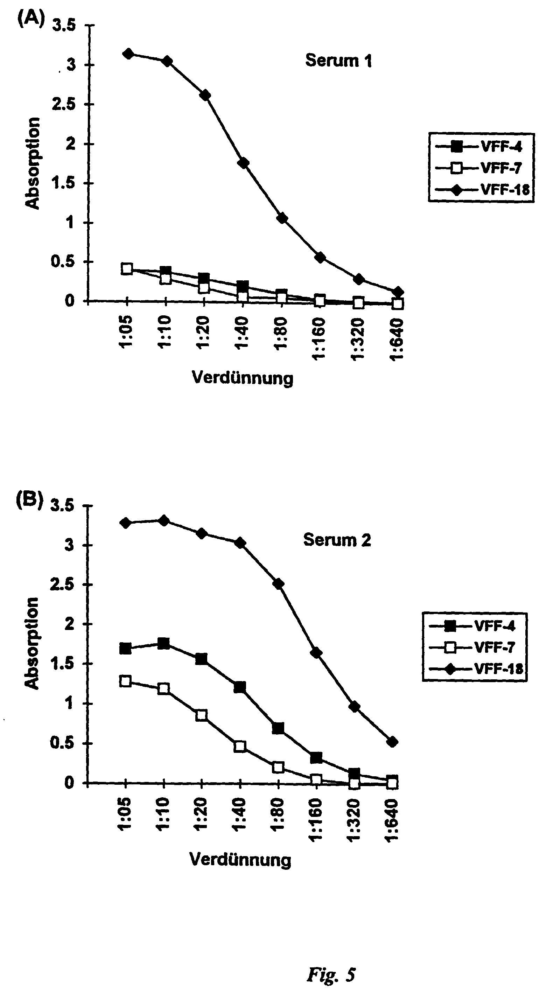

***Figur 5:*** Nachweis von löslichen CD44-Varianten, die das Exon v6 enthalten, in humanem Normalserum. In einem Sandwich-ELISA wurden die drei v6-spezifischen Antikörper VFF-4, VFF-7 bzw. VFF-18 als Beschichtungsantikörper eingesetzt. In allen drei Fällen wurde als Nachweisantikörper ein mit Peroxidase konjugierter CD44std-spezifischer Antikörper (BU-52, std = standard) verwendet. Gezeigt ist das Signal von zwei verschiedenen humanen Normalseren bei unterschiedlichen Verdünnungen in diesen Tests (***(A)*** und ***(B)***). In beiden Fällen ist mit VFF-18 ein wesentlich stärkeres Signal zu beobachten als mit den beiden anderen Antikörpern.

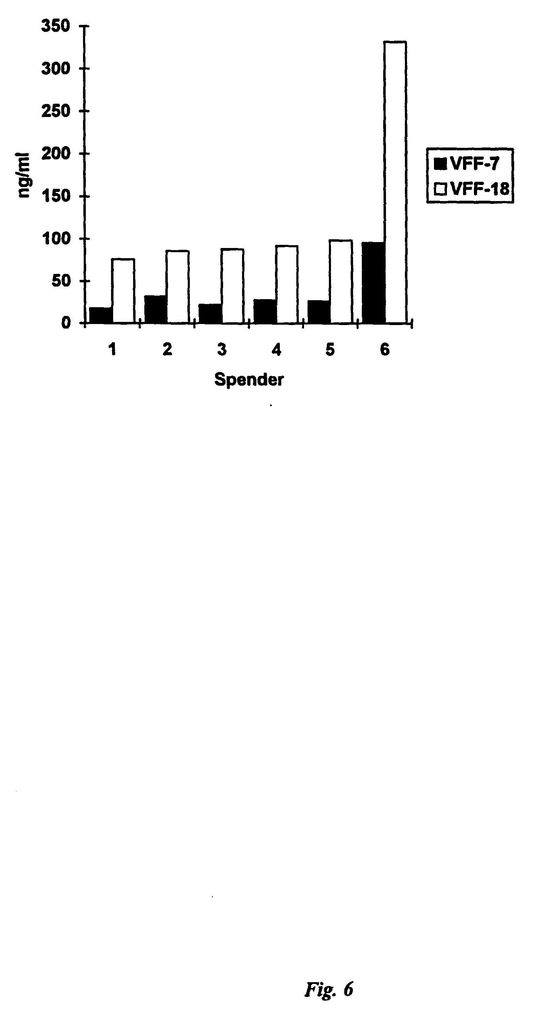

***Figur 6:*** Serumwerte von v6 enthaltenden CD44-Varianten. Mit zwei verschiedenen ELISAs wurde der Gehalt an löslichem CD44var in Seren von 6 gesunden Spendern bestimmt. In einem Test wird VFF-7, im anderen VFF-18 als Beschichtungsantikörper verwendet; beide Antikörper erkennen das Exon v6. Als Vergleichspräparat diente in beiden Fällen eine in CHO-Zellen hergestellte rekombinante lösliche CD44-Variante (Exon v3-v10). Der VFF-18-ELISA zeigt im Durchschnitt um 3.5mal höhere Werte an als der VFF-7-ELISA. Dies bedeutet, daß das im Serum vorkommende lösliche CD44var von VFF-18 besser erkannt wird als von VFF-7.

### Beispiele

### Beispiel 1: Herstellung des monoklonalen Antikörpers VFF-18

### Klonierung von pGEX-Fusionsproteinen

Die gesamte variante Region des HPKII-Typs von CD44v (Hofmann *et al*., 1991) wurde aus menschlicher Keratinozyten-cDNA durch Polymerase-Kettenreaktion (PCR) amplifiziert. Die beiden PCR-Primer *5*'-CAGGCTGGGAGCCAAATGAAGAAAATG-*3*', Positionen 25-52, und *5*'-TGATAAGGAACGATTGACATTAGAGTTGGA-*3*', Positionen 1013-984 der LCLC97-varianten Region, wie von Hofmann *et al*., beschrieben, enthielten eine *Eco*RI-Erkennungsstelle, die benutzt wurde, um das PCR-Produkt direkt in den Vektor pGEX-2T (Smith *et al*., 1988) zu klonieren. Das resultierende Konstrukt (pGEX CD44v HPKII, v3-v10) kodiert für ein Fusionsprotein von ∼70 kD, bestehend aus Glutathion-S-transferase von *Schistosoma japonicum* und den Exons v3-v10 von humanem CD44 (Fig. 1; Heider *et al*., 1993a). Das Fusionsprotein wurde in *E. coli* exprimiert und anschließend über Glutathion-Agarose affinitätsgereinigt (Smith *et al*., 1988).

### Immunisierung und Screening

Weibliche Balb/c Mäuse wurden intraperitoneal mit dem affinitätsgereinigten Fusionsprotein nach folgendem Schema immunisiert:
1. Immunisierung: 90 µg Fusionsprotein in komplettem Freund'schen Adjuvans
2. und 3. Immunisierung: 50 µg Fusionsprotein in inkomplettem Freund'schen Adjuvans.

Die Immunisierungen erfolgten im Abstand von jeweils 4 Wochen. 14 Tage nach der letzten Immunisierung wurden die Tiere noch an drei aufeinanderfolgenden Tagen mit jeweils 10 µg Fusionsprotein in PBS immunisiert. Am darauffolgenden Tag wurden Milzzellen eines Tieres mit hohem Antikörpertiter mit P3.X63-Ag8.653-Mausmyelomzellen mit Hilfe von Polyethylenglykol 4000 fusioniert. Die Hybridomzellen wurden dann in Mikrotiterplatten in HAT-Medium selektioniert (Köhler *et* Milstein, 1975; Kearney *et al*., 1979).

Die Bestimmung des Antikörpertiters im Serum bzw. das Screening der Hybridomüberstände wurde mit Hilfe eines ELISAs durchgeführt. Bei diesem Test wurden zunächst Mikrotiterplatten mit Fusionsprotein (GST-CD44v3-10) oder nur mit Glutathion-S-transferase beschichtet. Anschließend wurde mit seriellen Verdünnungen von Serumproben bzw. Hybridomüberständen inkubiert und die spezifischen Antikörper mit Peroxidase-konjugierten Antikörpern gegen Maus-Immunglobulin nachgewiesen. Hybridome, die nur mit Glutathion-S-transferase reagierten, wurden verworfen. Die verbleibenden Antikörper wurden zunächst in einem ELISA mit domänenspezifischen Fusionsproteinen (Exon v3, Exon v5 + v6, Exon v6 + v7, Exon v8 - v10) charakterisiert (Koopman *et al*., 1993). Ihre immunhistochemische Reaktivität wurde an menschlichen Hautschnitten getestet. Der Antikörper VFF-18 wurde dann über Bindung an das synthetische Peptid Hu1 (QWFGN RWHEG YRQT) identifiziert. Die Sequenz von Hu1 ist ein Fragment des humanen CD44-Exons v6.

### Beispiel 2: Bindung von CD44v6-spezifischen Antikörpern an synthetische Peptide

Die Bindung CD44v6-spezifischer Antikörper an synthetische Peptide wurde in einem ELISA bestimmt.

### Lösungen:

- Beschichtungspuffer:: 0.05 M Natriumcarbonat pH 9.6
- Assay-Puffer:: PBS (Phosphate Buffered Saline
0.5 % BSA (Rinderserumalbumin)
0.05 % Tween 20
- Substratlösung: Fa. Kierkegaard & Perry Laboratories, Gaithersburg MD, USA; TMB Peroxidase Substrat: Peroxidase Lösung B (H₂O₂) 1:1

Die Peptide (50 µg/ml in Beschichtungspuffer) wurden an NUNC Maxisorp Immunoplatten (1.1ASML) oder Acti-A-Platten der Fa. Bio Products (VFF-Antikörper) bei 4°C über Nacht immobilisiert. Bei den Acti-A-Platten wird das Peptid kovalent an die Platte gebunden. Anschließend wurde mit PBS/0.05% Tween gewaschen, freie Adsorptionsstellen an der Plattenoberfläche mit Assay-Puffer blockiert (1 Stunde bei Raumtemperatur), und wiederum einmal mit PBS/0.05% Tween 20 gewaschen. Acti-A-Platten wurden nach dem Blockieren mit 10 mM Natriumborhydrid in 20 mM Natriumhydrogencarbonat, pH 9.0, reduziert (1 Stunde bei Raumtemperatur schütteln) und anschließend dreimal mit PBS/0.05% Tween 20 gewaschen. Dann wurden Hybridomüberstände bzw. Antikörperlösungen in Assay-Puffer in Konzentrationen zwischen 0.02 und 10.0 µg/ml in die Näpfe gegeben und zwei Stunden bei Raumtemperatur in einem Plattenschüttler inkubiert. Daran schlossen sich drei Waschschritte mit PBS/0.05% Tween 20 an. Dann wurden 100 µl/Napf mit Meerrettichperoxidase konjugierter anti-Maus-IgG-Antikörper in einer geeigneten Verdünnung in Assay Puffer zugesetzt. Nach zweistündiger Inkubation bei Raumtemperatur am Plattenschüttler wurde dreimal mit PBS/0.05% Tween 20 gewaschen und mit Substratlösung gefärbt. Nach 10-15 min Entwicklung wurde mit 2 M Schwefelsäure gestoppt, und die Absorption bei 450 nm (Referenz 690 nm) am Photometer gemessen.

In einem ersten Experiment wurde die Bindung des für Ratten-CD44v6 spezifischen Antikörpers 1.1ASML an das Peptid Ra1 (KWFEN EWQGK NPPT) nachgewiesen (Tabelle 1, Figur 3 (A)).

**Tabelle 1**

| 1.1 ASML µg/ml | Absorption |
|---|---|
| 10.00 | 1.652 |
| 5.00 | 1.702 |
| 2.50 | 1.658 |
| 1.25 | 1.595 |
| 0.63 | 1.502 |
| 0.31 | 1.211 |
| 0.16 | 0.971 |
| 0.08 | 0.686 |
| 0.04 | 0.458 |
| 0.02 | 0.270 |

In einem weiteren Versuch wurde ein dem Ra1 homologes Peptid aus der humanen CD44v6 Sequenz synthetisiert (Hu1, QWFGN RWHEG YRQT). Verschiedene anti-human-CD44v6-Antikörper wurden an Hu1 gebunden. Dabei zeigt sich, daß VFF-18 eine überraschend höhere Bindungsaffinität aufweist als alle anderen eingesetzten Antikörper (Tabelle 2, Figur 3 (B)).

**Tabelle 2**

| Antikörper | Absorption |
|---|---|
| VFF-2 | 0.067 |
| VFF-4 | 1.333 |
| VFF-7 | 0.199 |
| VFF-18 | 2.384 |

Zur quantitativen Evaluierung wurden außerdem gereinigte anti-human-CD44v6-Antikörper in verschiedenen Konzentrationen an das Peptid Hu1 gebunden. Auch hier zeigt sich die deutlich bessere Bindungsaffinität im Vergleich mit den anderen Antikörpern (Tabelle 3, Figur 3 (C))

**Tabelle 3**

| µg/ml Antikörper | VFF-4 | VFF-7 | VFF-18 |
|---|---|---|---|
| 10.000 | 1.225 | 0.247 | 2.320 |
| 3.333 | 1.404 | 0.226 | 2.550 |
| 1.111 | 0.853 | 0.094 | 2.483 |
| 0.370 | 0.336 | 0.054 | 2.426 |
| 0.123 | 0.163 | 0.028 | 1.354 |
| 0.041 | 0.086 | 0.025 | 0.615 |
| 0.014 | 0.048 | 0.021 | 0.266 |
| 0.005 | 0.036 | 0.031 | 0.095 |
| 0.002 | 0.021 | 0.021 | 0.061 |

### Beispiel 3: Bindung radioaktiv markierter CD44v6-specifischer Antikörper an Tumorzellinien

### Radioaktive Markierung der Antikörper

1 mCi N-Succinimidyl-[2,3-³H]-propionat ([³H]-NSP, Amersham 1 mCi/ml) wurden in einem silikonisierten Probenröhrchen bei 0°C am Wasserstrahlvakuum bis fast zur Trockenen eingedampft. 15 µg Antikörper (1 mg/ml in PBS, pH 7,4) wurden zugesetzt und 48 Stunden lang bei 4°C inkubiert. Anschließend wurde überschüssiges [³H]-NSP durch Reaktion mit 30µl 1M Glycin in PBS (20 min bei Raumtemperatur) abgefangen. Die Abtrennung des markierten Antikörpers vom [³H]-Glycin erfolgte über eine Sephadex-G-25-M-Säule (Säulenvolumen 15 ml), wobei als Laufpuffer PBS/0.5% BSA verwendet wurde. Der [³H]-markierte Antikörper erscheint im Ausschlußvolumen. Die Menge an Antikörper wurde in einem ELISA zur Detektion von Mäuseimmunglobulin bestimmt und die spezifische Aktivität errechnet.

### Bindung der radiomarkierten Antikörper an Tumorzellen

Die drei Antikörper VFF-7 (anti-v6), VFF-8 (anti-v5) und VFF-18 (anti-v6) wurden mit N-Succinimidyl-[2,3-³H]propionat radioaktiv markiert und für Bindungstests an verschiedenen Tumorzellinien eingesetzt. Folgende Zellinien wurden dabei verwendet: CHO-CD44var: rekombinante Hamsterzellinie (Chinese Hamster Ovary), die humanes variantes CD44 (Exons v3-v10) an der Oberfläche exprimiert; HCT-116, CX-1, HT-29, CaCo, COLO 205: humane Kolonkarzinom-Linien; A431: humane Plattenepithelkarzinom-Linie.

Die Zellen wurden in 12-Loch-Gewebekulturplatten angesetzt, über Nacht bei 37°C im CO₂-Brutschrank inkubiert, anschließend einmal mit PBS gewaschen und mit Ethanol fixiert (1 min. bei Raumtemperatur). Dann wurde einmal mit Kulturmedium (RPMI 1640/10% fötales Kälberserum) gewaschen und der radioaktive Antikörper (250 000 dpm/Napf in Kulturmedium) gebunden. Nach einer Inkubation von 25 Stunden bei Raumtemperatur am Plattenschüttler wurde dreimal mit PBS/0.5 % BSA gewaschen, die Zellen mit 0.1 M NaOH/1% Triton X-100 solubilisiert und die Radioaktivität im Szintillationszähler gemessen. Unspezifische Bindung wurde in Anwesenheit von einem 100fachen Überschuß unmarkiertem Antikörper bestimmt. Die Bindung wurde auf eine einheitliche Zellzahl (400 000 Zellen) bezogen. Nach Bestimmung der spezifischen Aktivität der Antikörper kann die gebundene Antikörpermenge in fmol ausgedrückt werden.

Tabelle 4 und Figur 4 zeigen die spezifische Bindung der Antikörper an die verschiedenen Zellinien. Während die Bindung der v6-spezifischen Antikörper VFF-7 und VFF-18 an die rekombinante Zellinie CHO-CD44var annähernd gleich ist, zeigen die Antikörper an den Tumorzellinien sehr unterschiedliches Bindungsverhalten. In einigen Fällen beobachtet man nur VFF-18- und in geringerem Umfang VFF-8-Bindung (HT-29, CaCo, COLO 205), bei den anderen Zellinien bindet VFF-18 wesentlich besser als VFF-7.

**Tabelle 4**

| Zellinie | VFF-7 fmol | VFF-8 fmol | VFF-18 fmol |
|---|---|---|---|
| CHO CD44var | 33.50 | 68.42 | 46.52 |
| HCT-116 | 1.44 | 11.08 | 20.22 |
| CX-1 | 0.19 | 2.20 | 9.48 |
| HT-29 | 0.00 | 2.60 | 26.07 |
| CaCo | 0.03 | 2.81 | 12.57 |
| COLO 205 | 0.00 | 0.32 | 2.88 |
| A431 | 8.19 | 42.32 | 38.73 |

### Beispiel 4: ELISA zur Bestimmung von löslichem CD44v6 im Serum

### Lösungen:

- Beschichtungspuffer:: 0.05 M Natriumcarbonat pH 9.6
- Assay-Puffer:: PBS (Phosphate Buffered Saline
0.5 % BSA (Rinderserumalbumin)
0.05 % Tween 20
- Sample Diluent: Fa. Bender MedSystems, Wien, Österreich
- Substratlösung: Fa. Kierkegaard & Perry Laboratories, Gaithersburg MD, USA; TMB Peroxidase Substrat: Peroxidase-Lösung B (H₂O₂) 1:1

Mikrotiterplatten (Nunc-Immunoplate MaxiSorp F96) wurden mit 5 µg/ml eines der CD44v6-spezifischen Antikörper beschichtet (Inkubation bei 4°C über Nacht). Anschließend wurde mit PBS/0,05% Tween gewaschen, freie Adsorptionsstellen an der Plattenoberfläche mit Assay-Puffer blockiert (1 Stunde bei Raumtemperatur), und wiederum einmal mit PBS/0.05% Tween 20 gewaschen. Die Serumproben wurden dann mindestens 1:5 in Sample Diluent vorverdünnt und in seriellen 1:2 Verdünnungen in der Platte in Sample Diluent weiterverdünnt. Anschließend wurden 50 µl/Napf mit Meerrettichperoxidase konjugierter anti-CD44std Antikörper (Klon BU-52, The Binding Site, Birmingham) in einer geeigneten Verdünnung (1:3000 - 1:10 000) in Assay-Puffer zugesetzt. Nach dreistündiger Inkubation bei Raumtemperatur am Plattenschüttler wurde dreimal mit PBS/0.05% Tween 20 gewaschen und mit Substratlösung gefärbt. Nach 10-15 min Entwicklung wurde mit 2M Schwefelsäure gestoppt, und die Absorption bei 450 nm (Referenz 690 nm) am Photometer gemessen.

Zur Quantifizierung wurde eine serielle Verdünnung in Assay-Puffer eines löslichen CD44-Standardpräparates parallel zu den Serumproben aufgelegt. Dieses Präparat wurde aus einem Überstand von rekombinanten Hamster Zellen (CHO) aufgereinigt, die lösliches CD44v3-v10 exprimieren. Bei CD44v3-v10 handelt es sich um eine humane CD44-Variante, die durch die Exons v3 bis v10 kodierte Peptidsequenzen enthält.

Tabelle 5 und Figur 5 zeigen den Nachweis von löslichen CD44-Varianten, die das Exon v6 enthalten, in humanem Normalserum. In einem Sandwich-ELISA wurden die drei v6-spezifischen Antikörper VFF-4, VFF-7 bzw. VFF-18 als Beschichtungsantikörper eingesetzt. In allen drei Fällen wurde als Nachweisantikörper ein mit Peroxidase konjugierter CD44std-spezifischer Antikörper (BU-52, std = standard) verwendet. Gezeigt ist das Signal von zwei verschiedenen humanen Normalseren bei unterschiedlichen Verdünnungen in diesen Tests. In beiden Fällen (***(A)*** und ***(B)***) ist mit VFF-18 ein wesentlich stärkeres Signal zu beobachten als mit den beiden anderen Antikörpern.

**Tabelle 5 A**

| *Serum 1* | | | |
|---|---|---|---|
| Verdünnung | VFF-4 | VFF-7 | VFF-18 |
| 1:5 | 0.406 | 0.417 | 3.143 |
| 1:10 | 0.378 | 0.289 | 3.055 |
| 1:20 | 0.296 | 0.179 | 2.630 |
| 1:40 | 0.207 | 0.072 | 1.778 |
| 1:80 | 0.109 | 0.062 | 1.084 |
| 1:160 | 0.050 | 0.030 | 0.594 |
| 1:320 | 0.026 | 0.008 | 0.318 |
| 1:640 | 0.012 | 0.007 | 0.159 |

**Tabelle 5 B**

| *Serum 2* | | | |
|---|---|---|---|
| Verdünnung | VFF-4 | VFF-7 | VFF-18 |
| 1:5 | 1.690 | 1.280 | 3.290 |
| 1:10 | 1.756 | 1.185 | 3.321 |
| 1:20 | 1.564 | 0.857 | 3.163 |
| 1:40 | 1.213 | 0.468 | 3.050 |
| 1:80 | 0.699 | 0.208 | 2.537 |
| 1:160 | 0.336 | 0.061 | 1.666 |
| 1:320 | 0.138 | 0.014 | 0.988 |
| 1:640 | 0.054 | 0.018 | 0.546 |

Tabelle 6 und Figur 6 zeigen Serumwerte von v6 enthaltenden CD44-Varianten. Mit zwei verschiedenen ELISAs wurde der Gehalt an löslichem CD44var in Seren von 6 gesunden Spendern bestimmt. In einem Test wurde VFF-7, im anderen VFF-18 als Beschichtungsantikörper verwendet; beide Antikörper erkennen das Exon v6. Als Vergleichspräparat diente in beiden Fällen eine in CHO-Zellen hergestellte rekombinante lösliche CD44-Variante (Exon v3-v10). Der VFF-18-ELISA zeigt im Durchschnitt um 3,5 mal höhere Werte an als der VFF-7-ELISA. Dies bedeutet, daß das im Serum vorkommende lösliche CD44var im Vergleich zum rekombinanten Protein von VFF-18 besser erkannt wird als von VFF-7.

**Tabelle 6**

| Spender | VFF-7 ng/ml | VFF-18 ng/ml | VFF-18/VFF-7 |
|---|---|---|---|
| 1 | 18.2 | 75.9 | 4.17 |
| 2 | 32.1 | 85.5 | 2.66 |
| 3 | 22.4 | 87.6 | 3.91 |
| 4 | 27.7 | 91.6 | 3.31 |
| 5 | 26.8 | 98.3 | 3.67 |
| 6 | 95.4 | 332 | 3.48 |
| Mittelwert | | | 3.53 |
| SD | | | 0.52 |
| CV% | | | 14.9% |

### Literatur

Barbas C F, Björling E, Chiodi F, Dunlop N, Cababa D, Jones T M, Zebedee S L, Persson M A A, Nara P L, Norrby E, Burton D R. Recombinant human Fab fragments neutralize human type 1 immunodeficiency virus *in vitro*. *Proc*. *Natl*. *Acad*. *Sci*. *U*.*S*.*A*. *89*: 9339-9343 (1992).
Breitz H B, Weiden P L, Vanderheyden J-L, Appelbaum J W, Bjorn M J, Fer M F, Wolf S B, Ratcliff B A, Seiler C A, Foisie D C, Fisher D R, Schroff R W, Fritzberg A R, Abrams P G. Clinical experience with rhenium-186-labeled monoclonal antibodies for radioimmunotherapy: results of phase I trials. *J*. *Nucl*. *Med*. *33*: 1099-1112 (1992).
Catty, D., Raykundalia, C. ELISA and related immunoassays. In: Catty, D (Hrsg). *Antibodies Vol*. *II*. IRL Press Oxford (1989), 97-152, s. S. 105-109.
Catty, D., Murphy, G. Immunoassays using radiolabels. In: Catty, D (Hrsg). *Antibodies Vol*. *II*. IRL Press Oxford (1989), 77-96.
Chatal J-F, Saccavini J-C, Gestin J-F, Thédrez P, Curtet C, Kremer M, Guerreau D, Nolibé D, Fumoleau P, Guillard Y. Biodistribution of indium-111-labeled OC 125 monoclonal antibody intraperitoneally injected into patients operated on for ovarian carcinomas. *Cancer Res*. *49*: 3087-3094 (1989).
Chaudhary V K, Batra J K, Galdo M G, Willingham M C, Fitzgerald D J, Pastan I. A rapid method of cloning functional variable-region antibody genes in *Escherichia coli* as single-chain immunotoxins. *Proc*. *Natl*. *Acad*. *Sci*. *U*.*S*.*A*. *87:* 1066 (1990).
Colcher D, Esteban J, Carrasquillo J A, Sugarbaker P, Reynolds J C, Bryant G, Larson S M, Schlom J. Complementation of intracavitary and intravenous administration of a monoclonal antibody (B72.3) in patients with carcinoma. *Cancer Res*. *47:* 4218-4224 (1987).
Coloma M J, Hastings A, Wims L A, Morrison S L. Novel vectors for the expression of antibody molecules using variable regions generated by polymerase chain reaction. *J*. *Immunol*. *Methods 152:* 89-104 (1992).
Friedmann P N, McAndrew S J, Gawlak S L, Chace D, Trail P A, Brown J P, Siegall C B. BR96 sFv-PE40, a potent single-chain immunotoxin that selectively kills carcinoma cells. *Cancer Res*. *53:* 334-339 (1993).
Goodwin D A. A new appoach to the problem of targeting specific monoclonal antibodies to human tumors using anti-hapten chimeric antibodies. *J*. *Nucl*. *Med*. *Biol*. *16:* 645 (1989).
Günthert, U., Hofmann, M., Rudy, W., Reber, S., Zöller, M., Haußmann, I., Matzku, S., Wenzel, A., Ponta, H., and Herrlich, P. A new variant of glycoprotein CD44 confers metastatic potential to rat carcinoma cells. *Cell 65:* 13-24 (1991).
Guesdon, J. I., Ternynck, T., Avrameas, S. *J*. *Histochem*. *Cytochem*. *27:* 1131 (1979).
Güssow D, Seemann G. Humanization of monoclonal antibodies. *Methods Enzymol*. *203:* 99-121 (1991):
Heider, K.-H., Hofmann, M., Horst, E., van den Berg, F., Ponta, H., Herrlich, P., and Pals, S.T. A human homologue of the rat metastasis-associated variant of CD44 is expressed in colorectal carcinomas and adenomatous polyps. *J*. *Cell Biol*. *120:* 227-233 (1993a).
Heider, K-H., Dämmrich, J., Skroch-Angel, P., Müller-Hermelink, H-K., Vollmers, H-P., Herrlich, P., and Ponta, H. Differential expression of CD44 splice variants in intestinal- and diffuse-type human gastric carcinomas and normal gastric mucosa. *Cancer Res*. *53:* 4197-4203 (1993b).
Hofmann, M., Rudy, W., Zöller, M., Tölg, C., Ponta, H., Herrlich P., and Günthert, U. CD44 splice variants confer metastatic behavior in rats: homologous sequences are expressed in human tumor cell lines. *Cancer Res*. *51:* 5292-5297 (1991).
Johnson, G. D. Immunofluorescence. In: Catty, D (Hrsg). *Antibodies Vol*. *II*. IRL Press Oxford (1989), 179-200, s. S. 180-189.
Johnson S, Bird R E. Construction of single-chain derivatives of monoclonal antibodies and their production in *Escherichia coli*. *Methods Enzymol*. *203:* 88-98 (1991).
Kearney, J.F., Radbruch A., Liesegang B., Rajewski K. A new mouse myeloma cell line that has lost imunoglobulin expression but permits construction of *anti*body-secreting hybrid cell lines. *J*. *Immunol*. *123:* 1548 (1979).
Keenan A.M, Weinstein J N, Carrasquillo J A, Bunn P A, Reynolds J C, Foon K A *et al*. Immunolymphoscintigraphy and the dose dependence of ¹¹¹In-labeled T101 monoclonal antibody in patients with cutaneous T-cell lymphoma. *Cancer Res*. *47:* 6093-6099 (1987).
Köhler, G., Milstein, C. Continous culture of fused cells secreting antibody of predefined specifity. *Nature 265:* 495 (1975)
Koopman, G., Heider; K.-H., Horts, E., Adolf, G. R, van den Berg, F., Ponta, H., Herrlich, P., Pals, S. T. Activated human lymphocytes and aggressive Non-Hodgkin's lymphomas express a homologue of the rat metastasis-associated variant of CD44. *J*. *Exp*. *Med*. *177:* 897-904 (1993).
Kreitman R J Hansen H J, Jones A L, FitzGerald D J P, Goldenberg D M, Pastan I. *Pseudomonas* exotoxin-based immunotoxins containing the antibody LL2 or LL2-Fab' induce regression of subcutaneous human B-cell lymphoma in mice. *Cancer Res*. *53:* 819-825 (1993).
Larson S M, Cheung N-K V, Leibel S A. Radioisotope Conjugates. *In:* DeVita V T, Hellman S, Rosenberg S A (Hrsg.). Biologic therapy of cancer. J. B. Lippincott Comp., Philadelphia, 496-511 (1991).
Mulshine J L, Magnani J L, Linnoila R I: Applications of monoclonal antibodies in the treatment of solid tumors. *In:* DeVita V T, Hellman S, Rosenberg S A (Hrsg.). Biologic therapy of cancer. J. B. Lippincott Comp., Philadelphia, 563-588 (1991).
Nesbit M, Fu Z F, McDonald-Smith J, Steplewski Z, Curtis P J. Production of a functional monoclonal antibody recognizing human colorectal carcinoma cells from a baculovirus expression system. *J*. *Immunol*. *Methods 151:* 201-208 (1992).
Perkins A C, Pimm M V. A role for gamma scintigraphy in cancer immunology and immunotherapy. *Eur*. *J*. *Nucl*. *Med*. *19:* 1054-1063 (1992).
Press O W, Eary J F, Badger C C, Martin P J, Appelbaum F R, Levy R, Miller R, Brown S, Nelp W B, Krohn K A, Fisher D, DeSantes K, Porter B, Kidd P, Thomas E D, Bernstein I D. Treatment of refractory Non-Hodgkin's lymphoma with radiolabeled MB-1 (anti-CD37) antibody. *J*. *Clin*. *Oncol*. *7:* 1027-1038 (1989).
Rudy, W., Hofmann, M., Schwartz-Albiez, R., Zöller, M., Heider, K.-H., Ponta, H., Herrlich, P. The two major CD44 proteins expressed on a metastatic rat tumor cell line are derived from different splice variants: Each one individually suffices to confer metastatic behaviour. *Cancer Res*. *53:* 1262-1268 (1993).
Schrappe M, Bumol T F, Apelgren L D, Briggs S L, Koppel G A, Markowitz D D, Mueller B M, Reisfeld R A. Long-term growth suppression of human glioma xenografts by chemoimmunoconjugates of 4-desacetylvinblastine-3-carboxyhydrazide and monoclonal antibody 9.2.27. *Cancer Res*. *52:* 3838-3844 (1992).
Screaton, G.R., Bell, M.V., Jackson, D.G., Cornelis, F.B., Gerth, U., and Bell, J. I. Genomic structure of DNA encoding the lymphocyte homing receptor CD44 reveals at least 12 alternatively spliced exons. *Proc*. *Natl*. *Acad*. *Sci*. *U*.*S*.*A*. *89:* 12160-12164 (1992).
Sears H F, Mattis J, Herlyn D, Häyry P, Atkinson B, Ernst C, Steplewski Z, Koprowski H. Phase-I clinical trial of monoclonal antibody in treatment of gastrointestinal tumours. *Lancet 1982 (1):* 762-765 (1982).
Seiter, S., Arch, R., Reber, S., Komitowski, D., Hofmann, M., Ponta, H:, Herrlich, P., Matzku, S., Zöller, M. Prevention of tumor metastasis formation by anti-variant CD44. *J*. *Exp*. *Med*. *177:* 443-455 (1993).
Senter P D, Schreiber G J, Hirschberg D L, Ashe S A, Hellström K E, Hellström I. Enhancement of the *in vitro* and *in vivo* antitumor activities of phosphorylated mitomycin C and etoposide derivatives by monoclonal antibody-alkaline phosphatase conjugates. *Cancer Res*. *49:* 5789-5792 (1989).
Shin S-U, Morrison S L. Production and properties of chimeric antibody molecules. *Methods Enzymol*. *178:* 459-476 (1989).
Siccardi A G, Buraggi G L, Callegaro L, Colella A C, DeFilippi P G *et al*. Immunoscintigraphy of adenocarcinomas by means of radiolabeled F(ab')₂ fragments of an anti-carcino-embryonic antigen monoclonal antibody: a multicenter study. *Cancer Res*. *49:* 3095-3103 (1989).
Smith, D.B., Johnson, K.S. Single-step purification of polypetides expressed in *Escherichia coli* as fusions with glutathione S-transferase. *Gene 67:* 31-40 (1988).
Srivastava S C (Hrsg). Radiolabeled monoclonal antibodies for imaging and therapy. *Life Sciences Series A 152*, Plenum New York (1988).
Tölg, C., Hofmann, M., Herrlich, P., and Ponta, H. Splicing choice from ten variant exons establishes CD44 variability. *Nucleic Acids*. *Res*. *21:* 1225-1229 (1993).
Theuer C P, Kreitman R J, FitzGerald D J, Pastan I. Immunotoxins made with a recombinant form of *pseudomonas* exotoxin A that do not require proteolysis for activity. *Cancer Res*. *53:* 340-347 (1993).
Thomas G D, Dykes P W, Bradwell A R. Antibodies for tumour immunodetection and methods for antibody radiolabeling. *In:* Catty D (Hrsg.). Antibodies. IRL Press Oxford, 223-244 (1989), 223-244.
Thompson C H, Stacker S A, Salehi N, Lichtenstein M, Leyden M J, Andrews J T. Immunoscintigraphy for detection of lymph node metastases from breast cancer. *Lancet* ***1984*** *(2):* 1245-1247 (1984).
Vitetta E S, Thorpe P E. Immunotoxins. In: DeVita V T, Hellman S, Rosenberg S A (Hrsg.). Biologic therapy of cancer. J. B. Lippincott Comp., Philadelphia, 482-495 (1991)
Vitetta E S, Stone M, Amlot P, Fay J, May R, Till M, Newman J, Clark P, Collins R, Cunningham D, Ghetie V, Uhr J W, Thorpe P E. Phase I immunotoxin trial in patients with B-cell lymphoma. *Cancer Res*. *51:* 4052-4058 (1991).
Wang S-M, Chem J-W, Yeh M-Y, Ng J C, Tung E, Roffler S R. Specific activation of glucuronide prodrugs by antibody-targeted enzyme conjugates for cancer therapy. *Cancer Res*. *52:* 4484-4491 (1992).
Weiner L M, O'Dwyer J, Kitson J, Comis R L, Frankel A E, Bauer R J, Kopnrad M S, Groves E S. Phase I evaluation of an anti-breast carcinoma monoclonal antibody 260F9-recombinant ricin A chain immunoconjugate. *Cancer Res*. *49:* 4062-4067 (1989).
Wielenga, V. J. M., Heider, K.-H., Offerhaus, G. J. A., Adolf, G. R., van den Berg, F. M., Ponta, H., Herrlich, P., Pals, S.T. Expression of CD44 variant proteins in human colorectal cancer is related to tumor progression. *Cancer Res*. *53:* 4754-4756 (1993).
Winter, G., Griffith, A. D., Hawkins, R. E., Hoogenboom, H. R. Making antibodies by phage display technology. *Ann*. *Rev*. *Immunol*. *12*, 433-455 (1994).

## Patentansprüche

1. Antikörper gegen ein Epitop innerhalb der Aminosäuresequenz, die durch das variable Exon v6 des CD44-Gens kodiert wird, dadurch gekennzeichnet, daß es der Antikörper VFF-18 ist, der von der Hybridoma-Zellinie mit der Hinterlegungsnummer DSM ACC2174 gebildet wird.

2. Hybridoma-Zellinie mit der Hinterlegungsnummer DSM ACC2174.

3. Antikörpermolekül, erhältlich durch chemische oder enzymatische Modifikation eines Antikörpers gemäß Anspruch 1.

4. Antikörpermolekül nach Anspruch 3, dadurch gekennzeichnet, daß es ein Fragment eines Antikörpers gemäß Anspruch 1 ist.

5. Antikörpermolekül nach Anspruch 4, dadurch gekennzeichnet, daß es ein Fab- oder ein F(ab')₂-Fragment ist.

6. Antikörpermolekül nach Anspruch 3, dadurch gekennzeichnet, daß der Antikörper mit einem anderen Molekül verknüpft ist.

7. Antikörpermolekül nach Anspruch 6, dadurch gekennzeichnet, daß das andere Molekül ein Polypeptid ist.

8. Antikörpermolekül nach einem der Ansprüche 3 bis 5, dadurch gekennzeichnet, daß es mit einem radioaktiven Isotop verknüpft ist.

9. Rekombinantes Antikörpermolekül mit dem Idiotyp eines Antikörpers gemäß Anspruch 1.

10. Rekombinantes Antikörpermolekül nach Anspruch 9, dadurch gekennzeichnet, daß es ein chimäres, humanisiertes, Einzelketten(single chain)- oder durch chain shuffling erzeugtes Antikörpermolekül ist.

11. Rekombinantes Antikörpermolekül nach Anspruch 9 oder 10, dadurch gekennzeichnet, daß es mit einem anderen Molekül oder mit einem radioaktiven Isotop verknüpft ist.

12. Antikörpermolekül, das das gleiche Epitop erkennt wie der Antikörper gemäß Anspruch 1.

13. Verwendung eines Antikörpers oder Antikörpermoleküls gemäß einem der Ansprüche 1 oder 3 bis 12 zu diagnostischen Verfahren außerhalb des menschlichen oder tierischen Körpers.

14. Verwendung nach Anspruch 13, dadurch gekennzeichnet, daß das Verfahren ein Enzym-gekoppelter Immunoassay oder ein Radioimmunoassay ist.

15. Verwendung nach Anspruch 13, dadurch gekennzeichnet, daß das Verfahren ein immunhistochemisches Verfahren ist.

16. Antikörper oder Antikörpermolekül nach einem der Ansprüche 1 oder 3 bis 12 zur pharmazeutischen Verwendung.

17. Verwendung eines Antikörpers oder Antikörpermoleküls gemäß einem der Ansprüche 1 oder 3 bis 11 zur Herstellung eines Mittels für die Diagnostik von Tumoren *in vivo*.

## Claims

1. Antibody against an epitope within the amino acid sequence coded by the variable exon v6 of the CD44 gene, characterized in that it is the antibody VFF-18 produced by the hybridoma cell line with the accession number DSM ACC2174.

2. Hybridoma cell line with the accession number DSM ACC2174.

3. Antibody molecule, obtainable by the chemical or enzymatical modification of an antibody according to claim 1.

4. Antibody molecule according to claim 3, characterized in that it is a fragment of an antibody according to claim 1.

5. Antibody molecule according to claim 4, characterized in that it is a Fab or F(ab')₂ fragment.

6. Antibody molecule according to claim 3, characterized in that the antibody is linked to another molecule.

7. Antibody molecule according to claim 6, characterized in that the other molecule is a polypeptide.

8. Antibody molecule according to any one of claims 3 to 5, characterized in that it is linked to a radioactive isotope.

9. Recombinant antibody molecule with the idiotype of an antibody according to claim 1.

10. Recombinant antibody molecule according to claim 9, characterized in that it is a chimeric, humanised, or single chain antibody molecule, or that it is an antibody molecule generated by chain shuffling.

11. Recombinant antibody molecule according to claim 9 or 10, characterised in that it is linked to another molecule or to a radioactive isotope.

12. Antibody molecule recognizing the same epitope as the antibody according to claim 1.

13. Use of an antibody or antibody molecule according to any one of claims 1 or 3 to 12 in a diagnostic method outside the human or animal body.

14. Use according to claim 13, characterized in that the method is an enzyme-linked immunoassay or a radioimmunoassay.

15. Use according to claim 13, characterized in that the method is an immunohistochemical method.

16. Antibody or antibody molecule according to any one of claims 1 or 3 to 12 for pharmaceutical use.

17. Use of an antibody or antibody molecule according to any one of claims 1 or 3 to 11 for preparing an agent for the diagnosis of tumours *in vivo*.

## Revendications

1. Anticorps contre un épitope dans la séquence d'acides aminés qui est codée par l'exon variable v6 du gène de CD44, caractérisé en ce qu'il s'agit de l'anticorps VFF-18 qui est formé par la lignée cellulaire d'hybridome de numéro de dépôt DSM ACC2174.

2. Lignée cellulaire d'hybridome de numéro de dépôt DSM ACC2174.

3. Molécule d'anticorps qui peut être obtenue par modification chimique ou enzymatique d'un anticorps selon la revendication 1.

4. Molécule d'anticorps selon la revendication 3 caractérisée en ce qu'il s'agit d'un fragment d'un anticorps selon la revendication 1.

5. Molécule d'anticorps selon la revendication 4 caractérisée en ce qu'il s'agit d'un fragment Fab ou F(ab')₂.

6. Molécule d'anticorps selon la revendication 3 caractérisée en ce que l'anticorps est lié à une autre molécule.

7. Molécule d'anticorps selon la revendication 6 caractérisée en ce que l'autre molécule est un polypeptide.

8. Molécule d'anticorps selon l'une des revendications 3 à 5 caractérisée en ce qu'elle est liée à un isotope radioactif.

9. Molécule d'anticorps recombinée ayant l'idiotype d'un anticorps selon la revendication 1.

10. Molécule d'anticorps recombinée selon la revendication 9 caractérisée en ce qu'il s'agit d'une molécule d'anticorps chimère, humanisée, à chaîne unique (single chain) ou produite par chain shuffling.

11. Molécule d'anticorps recombinée selon la revendication 9 ou 10 caractérisée en ce qu'elle est liée à une autre molécule ou à un isotope radioactif.

12. Molécule d'anticorps qui reconnaît le même épitope que l'anticorps selon la revendication 1.

13. Utilisation d'un anticorps ou d'une molécule d'anticorps selon l'une des revendications 1 ou 3 à 12 pour des procédés diagnostiques à l'extérieur du corps humain ou animal.

14. Utilisation selon la revendication 13 caractérisée en ce que le procédé est un dosage immunoenzymatique ou un radioimmunodosage.

15. Utilisation selon la revendication 13 caractérisée en ce que procédé est un procédé immunohistochimique.

16. Anticorps ou molécule d'anticorps selon l'une des revendications 1 ou 3 à 12 pour l'utilisation pharmaceutique.

17. Utilisation d'un anticorps ou d'une molécule d'anticorps selon l'une des revendications 1 ou 3 à 11 pour la préparation d'un agent pour le diagnostic de tumeurs in vivo.
